# EUROPEAN PATENT APPLICATION

(11) **EP 3 819 629 A1**
(43) Date of publication of application: **12.05.2021**
(21) Application number: 19831001.3
(22) Date of filing: 28.06.2019
(51) Int. Cl.: G01N 21/3577

(54) **SENSOR COVER, SENSOR DEVICE PROVIDED WITH SAME, LIQUID PROPERTY MEASURING METHOD AND METHOD FOR PRODUCING METABOLITE IN AERATION CULTURE**

(30) Priority: 06.07.2018 JP 2018128836
(71) Applicant: AJINOMOTO CO., INC., Chuo-ku Tokyo 104-8315 (JP)
(72) Inventor: KODAMA, Yohei, Kawasaki-shi, Kanagawa 210-8681 (JP); FUJIE, Masaaki, Kawasaki-shi, Kanagawa 210-8681 (JP)
(74) Representative: Mewburn Ellis LLP
(86) International application number: PCT/JP2019/025828
(87) International publication number: WO 2020/009022

(57) **Abstract**

Provided is a sensor cover and a sensor apparatus including the same. The present invention is a cover (10) for a sensor for measuring properties of a solution in which gas bubbles are mixed, and has a cover main body (14) disposed to surround a sensor (12), wherein a lower transmitting portion for passing solution (14a) is disposed on at least a part of the surface of the bottom side of this cover main body (14), an upper transmitting portion for passing solution (14b) is disposed on at least a part of the upper surface of the cover main body (14), many fine holes for passing solution are formed on the lower and upper transmitting portions, and the diameter of fine holes placed on the lower transmitting portion (14a) is formed to be smaller than the diameter of the fine holes placed on the upper transmitting portion (14b).

## Description

### Technical Field

0001 The present invention pertains to a sensor cover, and more particularly to a cover for a sensor used to measure properties of a solution into which air is mixed, and a sensor apparatus including same.

### Background Art

0002 In various industrial production processes there is a need to measure properties of solutions such as the electrical conductivity or turbidity of the solution, or the concentration of specific components in the solution. Japanese Patent No. 3074781 (Patent Document 1) sets forth a method for manufacturing L-lysine. In this manufacturing method, a carbon source is added to maintain a carbon source concentration of 5g/L or less in a culture solution. I.e., in this method, a culture solution is sampled at appropriate times and its carbon source concentration directly measured, or its pH or dissolved oxygen concentration is measured to detect deficiencies of the carbon source using changes therein as a way of measuring carbon source concentration to control feeding of the medium.

0003 As noted in the invention set forth in the Patent Document 1, the properties of a solution can be measured relatively easily by removing a portion of the solution to be measured from the production process, but it is desirable from a manufacturing efficiency standpoint to measure in-line, without removing material from the manufacturing floor. However it can be difficult to measure solution properties, especially in-line, when gas bubbles mix into the solution to be measured. For example, bubbles of supplied oxygen or air, or bubbles of carbon dioxide gas occurring as the metabolic product of microorganisms in the culture, can become mixed into a culture solution while it is being aerobically cultured, thus adding noise to measurement values and increasing measurement errors.

0004 Japanese Patent No. 4420168 (Patent Document 2) sets forth a turbidity sensor. This turbidity sensor has a stainless steel hollow semicircular tube member, on the bottom portion of which a test fluid intake opening and an automatically opening and closing swing valve are disposed, and on the top portion of which a foam draining hole is formed. In addition, the solution-contacting light measuring portion of the laser turbidimeter is disposed on the tip position thereof, on the interior of the hollow semicircular tube. For measurements using this turbidity sensor, the swing valve is first opened and test fluid in the hollow semicircular tube is exchanged. The swing valve is then closed, gas bubbles inside the hollow semicircular tube are discharged from the foam drain hole, and turbidity is measured after waiting for the turbidity targeted for detection to stabilize. In the invention of Patent Document 2, the effects of gas bubbles in the fluid under test are in this manner reduced.

### Prior Art Documents

0005

### Patent Documents

Patent Document 1: Japanese Patent No. 3074781
Patent Document 2: Japanese Patent No. 4420168

### Summary of Invention

### Problems the Invention Seeks to Solve

0006 However, in the turbidity sensor set forth in Patent Document 2 it is necessary to wait for detection values to stabilize after the swing valve is closed, which makes real time detection difficult. Also, the turbidity sensor of Patent Document 2 has the problem that it requires a swing valve or the like which can be opened and closed by remote operation, therefore the structure is complicated, and maintenance to ensure stable operation over a long time period is burdensome.

0007 The present invention thus has the object of using a simple structure to provide a sensor cover capable of reducing the effects of gas bubbles mixed into a solution, and a sensor apparatus furnished with same.

### Means for Solving the Problem

0008 In order to resolve the above-described problems, the present invention is a sensor cover for a sensor for measuring properties of a solution into which gas bubbles are mixed, comprising: a cover main body disposed to surround the sensor; wherein a lower transmitting portion for passing solution is provided on at least a portion of a lower surface of the cover main body, and an upper transmitting portion for passing solution is provided on at least a portion of an upper surface of the cover main body; and a large number of fine holes for passing solution are respectively formed on the lower transmitting portion and the upper transmitting portion, wherein the fine holes provided on the lower transmitting portion are formed to be smaller than the fine holes provided on the upper transmitting portion.

0009 In the invention thus constituted, a lower transmitting portion and upper transmitting portion for passing solution are placed on a cover main body disposed to surround the sensor, therefore the solution to be measured can constantly flow in and out of the cover main body. As a result, solution within the cover main body is constantly exchanged, and the sensor disposed within the cover main body can continuously measure solution properties in real time. Also, the fine holes placed in the lower transmitting portion are formed to be smaller than the fine holes placed in the upper transmitting portion, therefore gas bubbles floating up from the bottom of the cover main body have difficulty passing through the fine holes on the lower transmitting portion, and gas bubbles can be effectively suppressed from penetrating into the cover main body. On the other hand, the fine holes in the upper transmitting portion are formed to be larger than the fine holes in the lower transmitting portion, therefore gas bubbles which have penetrated into the cover main body can easily float up within the cover main body to be discharged through the upper transmitting portion. As a result, accumulation in the cover main body of gas bubbles which have penetrated into the cover main body, and the resulting adverse influence on sensor measurement values, can be suppressed.

0010 In the present invention the cover main body is preferably formed in an approximately cylindrical shape, and the sensor extends in an axial direction on an interior of the cover main body.

In the invention thus constituted, the cover main body is formed in an approximately cylindrical shape, so gas bubbles which have floated up from the lower side of the cover main body can easily flow up along the lower wall surface of the cover main body, and gas bubbles can be further suppressed from penetrating into the cover main body. Since the cover main body is formed in an approximately cylindrical shape, gas bubbles which penetrate the cover main body and float upward are collected in the highest part within the cover main body, and have difficulty contacting the sensor. Adverse influence on measurement can thus be minimized, even if gas bubbles penetrate into the cover main body.

0011 In the present invention, preferably, the lower transmitting portion is placed on an entire surface of a lower semicircular portion of the approximately cylindrical cover main body, and the upper transmitting portion is placed on an entire surface of an upper semicircular portion of the cover main body.

In the invention thus constituted, the lower transmitting portion and upper transmitting portion are respectively placed over the entire surface of the lower semicircular portion and the upper semicircular portion, therefore the part of the cover main body through which solution passes can be made extremely large.

As a result, solution in the cover main body can be easily exchanged with outside solution, thus enabling accurate measurement of solution properties in real time.

0012 In the present invention, preferably, the cover main body is formed of a thin metal plate, and the fine holes in the lower transmitting portion and the upper transmitting portion are approximately round holes made in the thin metal plate.

In the invention thus constituted, fine holes in the lower transmitting portion and upper transmitting portion are formed by holes made in a metal thin plate, so compared to the case in which the lower transmitting portion or upper transmitting portion are formed of a mesh product formed by weaving strands of wire, solution has difficulty adhering to the lower transmitting portion or upper transmitting portion, and maintainability of the sensor cover is improved. Forming fine holes in the lower transmitting portion and upper transmitting portion as holes disposed in thin metal plate results in lower propensity to breakage than with mesh, enabling the formation of a high-durability cover main body.

0013 In the present invention, preferably, a diameter of the fine holes provided in the upper transmitting portion is between 1.5 and 5 times a diameter of the fine holes provided in the lower transmitting portion.

In the invention thus constituted, the diameter of fine holes disposed in the upper transmitting portion is set to between 1.5 and 5 times the diameter of fine holes disposed in the lower transmitting portion, thus suppression of penetration by gas bubbles into the cover main body can be appropriately balanced against the discharge of those gas bubbles which do penetrate into the cover main body, so the influence of gas bubbles on the sensor can be effectively suppressed.

0014 In the present invention, preferably, the diameter of fine holes in the lower transmitting portion is 100µm to 400µm, and more preferably 150µm to 400µm.

In the invention thus constituted, fine holes placed on the lower transmitting portion are formed at a diameter of 100µm to 400µm, therefore the penetration of gas bubbles of a size that could easily adversely influence sensor measurement values can be effectively suppressed, while solution is still permitted to flow into the cover main body.

0015 In the present invention, preferably, the cover main body is placed to project diagonally downward from a side wall surface of a container in which the solution is held, and a measurement portion of the sensor is disposed close to an end of the cover main body.

In the invention thus constituted, the cover main body projects diagonally downward from the side wall surface of the container in which the solution is held, therefore gas bubbles floating up from below and reaching the cover main body can easily flow upward along the bottom surface of the cover main body, and penetration into the cover main body can be effectively suppressed. Gas bubbles that have penetrated into the cover main body collect in the base end portion of the cover main body located above, traveling away from the measurement portion of the sensor disposed close to the end of the cover main body, reducing adverse influence on measurement.

0016 The sensor apparatus of the present invention comprises a sensor sensor for measuring properties of a solution; and the cover of any one of Claims 1 through 7, disposed to surround the sensor.

0017 In addition, the solution property measurement method of the present invention comprises a step for preparing the sensor apparatus of the present invention; and a step for acquiring a measurement signal from the sensor apparatus inserted into the solution.

The method of the present invention for manufacturing metabolic products in an aerobic culture comprises a step for preparing the sensor apparatus of the present invention a step for acquiring a measurement signal from the sensor apparatus inserted into a culture solution; a step for aerobic culturing in the culture solution; and a step for tracking changes in concentration of the metabolic products in the aerobic culture and/or changes in concentration of a culture raw material based on the measurement signal.

### Effect of the Invention

0018 Using the sensor cover and sensor apparatus comprising same of the present invention, the influence of gas bubbles blended in a solution can be reduced using a simple structure.

### Brief Explanation of Figures

0019
[Fig. 1] A cross section showing an example in which a sensor comprising a sensor cover according to an embodiment of the invention is applied to an amino acid fermenter.
[Fig. 2] A perspective view showing the external appearance of a sensor cover according to an embodiment of the invention.
[Fig. 3] A cross section showing an expanded view of a sensor apparatus according to an embodiment of the present invention, attached to the side wall surface of a fermenter.
[Fig. 4] A diagram showing an expanded view of an example of fine holes disposed on a cover main body surface in a sensor cover according to an embodiment of the present invention.
[Fig. 5] A diagram schematically depicting a comparison of the operation (5A, 5b) of conventional sensor covers with the operation of a sensor cover (5C) according to an embodiment of the present invention.
[Fig. 6] A diagram showing a comparative example of a case in which amino acid concentration is measured without using a sensor cover according to an embodiment of the present invention.
[Fig. 7] A diagram showing a comparative example in which amino acid concentration is measured using a conventional sensor cover.
[Fig. 8] A diagram showing an example of measuring amino acid concentration by a sensor apparatus using a sensor cover according to an embodiment of the present invention.
[Fig. 9] A diagram showing an example of measuring the concentration of amino acid in a culture solution different from that of Figs. 6 through 8, using a sensor cover according to an embodiment of the present invention.
[Fig. 10] A diagram showing an example of measuring the concentration of amino acid in a culture solution different from that of Figs. 6 through 8, using a sensor cover according to an embodiment of the present invention.

### Embodiments

0020 Next, referring to the attached figures, we explain preferred embodiments of the invention.

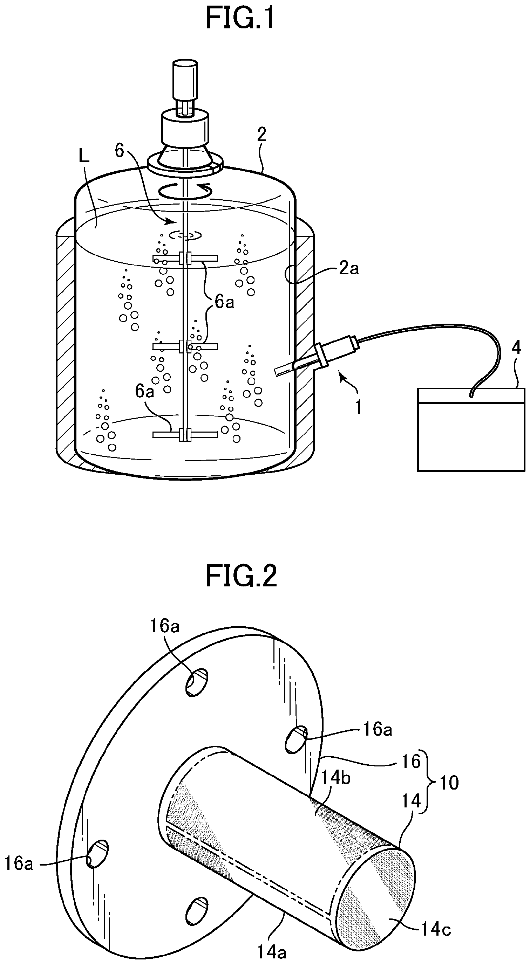

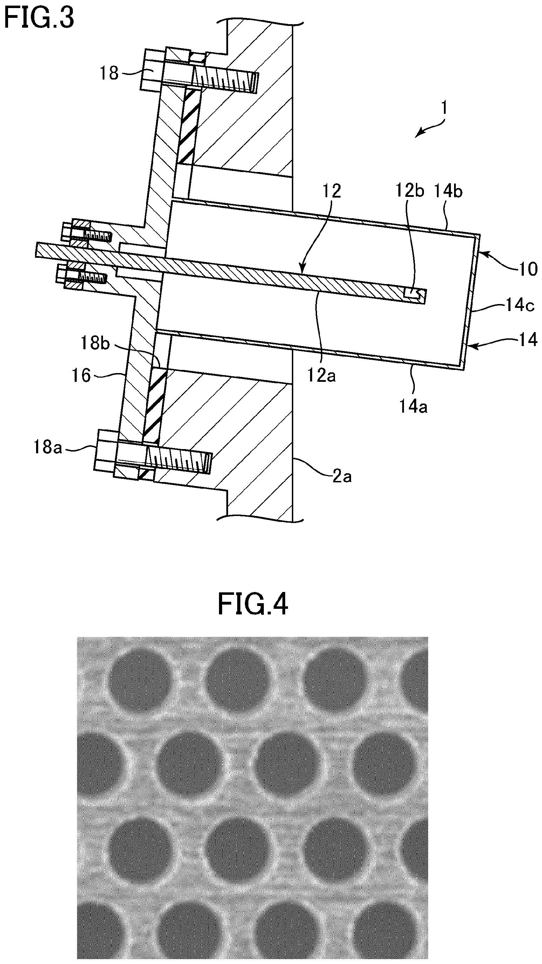

Fig. 1 is a cross section showing an example in which a sensor comprising a sensor cover according to an embodiment of the invention is applied to an amino acid fermenter. Fig. 2 is a perspective view showing the external appearance of a sensor cover according to an embodiment of the invention. Fig. 3 is a cross section showing an expanded view of a sensor apparatus according to an embodiment of the present invention attached to the side wall surface of a fermenter.

0021 As shown in Fig. 1, a sensor apparatus 1 comprising a sensor cover according to an embodiment of the present invention is disposed on side wall surface 2a of fermenter 2, which is a solution-holding container, and is constituted to perform in-line measurement of the concentration of amino acid in a culture solution L held in fermenter 2. In addition, a sensor is disposed on the interior of sensor apparatus 1, and a signal acquired by this sensor is transmitted to a measurement instrument main unit 4. In the present embodiment, fermenter 2 is approximately cylindrical, and a mixer 6 for stirring the culture solution is disposed along the center axis thereof. The mixer 6 comprises multiple blades 6a for mixing culture solution L; the culture solution L in fermenter 2 is mixed homogeneously by rotating these blades 6a. When aerobic culturing is performed in fermenter 2, oxygen or air is introduced into fermenter 2 from a supply device (not shown). Therefore fine gas bubbles of supplied oxygen or air, or fine gas bubbles of carbon dioxide gas produced by the microorganisms being cultured, are mixed into the culture solution L.

0022 Next, referring to Figs. 2 and 3, we explain the constitution of sensor apparatus 1 according to an embodiment of the present invention.

As shown in Fig. 3, sensor apparatus 1 has a sensor cover 10 according to an embodiment of the present invention, and a sensor 12 disposed on the interior of this sensor cover 10.

0023 The sensor cover 10 has an approximately cylindrical cover main body 14, and a flange portion 16 placed at the base end of this cover main body 14 and affixed to the side wall surface 2a of fermenter 2.

The cover main body 14 is formed of thin stainless steel plate, constituted as an approximately cylindrical shape, closed at one end, and disposed to surround sensor 12. As described below, many fine holes are disposed over the entire surface of cover main body 14 so that solution inside fermenter 2 can flow into cover main body 14 through these fine holes. Note that the cover main body can be constituted in non-cylindrical shapes such as a parallelepiped. The cover main body can also be formed of metal other than stainless steel, or of resins such as polytetrafluoroethylene, and is preferable formed of a material resistant to breakage by the flow of solution resulting from mixing.

0024 The flange portion 16 is a stainless steel disk; cover main body 14 is affixed to the center thereof by welding to form an integral piece. Also, cover main body 14 is perpendicularly attached to the flat surface of flange portion 16. A bolt hole 16a is formed in flange portion 16, and flange portion 16 is affixed to the outside of side wall surface 2a by an affixing bolt 18a. A packing 18b is disposed between flange portion 16 and side wall surface 2a to secure a watertight seal between flange portion 16 and side wall surface 2a. In addition, as shown in Fig. 3, the part which affixes flange portion 16 on the outside of side wall surface 2a is formed at a slope relative to vertical. Thus when sensor apparatus 1 is attached to the side wall surface 2a of fermenter 2, cover main body 14 projects diagonally downward on the inside of the container side wall. The angle at which cover main body 14 is attached is preferably set to both suppress the inflow of gas bubbles into cover main body 14 and to suppress the accumulation of solution near the cover main body 14 which leads to growth of miscellaneous bacteria; depending on properties of the solution, an angle close to horizontal can also be set. In the embodiment, the center axis of cover main body 14 is tilted by approximately 16 degrees relative to the horizontal axis.

0025 In the present embodiment a transmissive/reflective near-infrared spectral sensor (NIR sensor) is used for sensor 12; amino acid concentration in culture solution L is measured by near-infrared analysis. However the sensor cover 10 of the present embodiment can be applied to various types of sensors in addition to NIR sensors, such as spectral sensors using ultraviolet or visible light, other optical sensors, or electromagnetic sensors for measuring permittivity or conductance, and these sensors can be combined to constitute a sensor apparatus.

0026 As shown in Fig. 3, sensor 12 comprises a rod-shaped sensor probe 12a with a circular cross section, on the tip portion of which a measurement portion 12b is disposed. The sensor probe 12a extends into cover main body 14 through an opening disposed at the center of the flange portion 16 on sensor cover 10. The sensor probe 12a extends along the center axis of cover main body 14, and measurement portion 12b, disposed at the tip portion thereof, is positioned close to the end portion of cover main body 14. Note that in the present embodiment sensor probe 12a has an outside diameter of approximately 20 mm, and is surrounded by cover main body 14, which has an outside diameter of approximately 60 mm. It is thus preferable to provide a clearance of approximately 10 to approximately 30 mm between sensor probe 12a and the inside wall surface of cover main body 14.

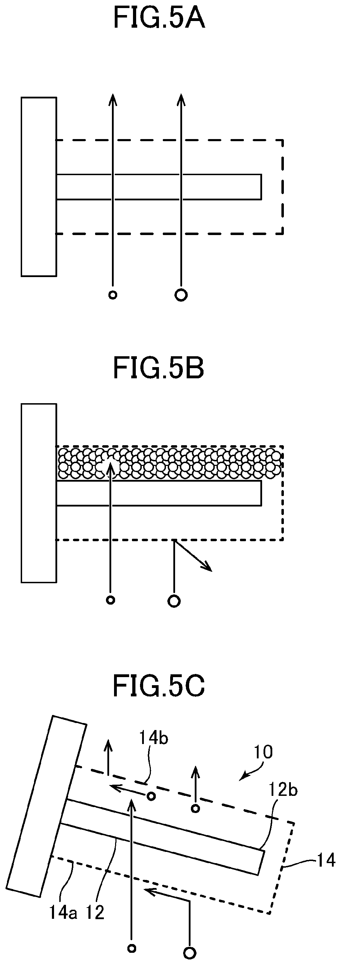

0027 Next, referring to Figs. 4 and 5, we explain the constitution of cover main body 14 provided on sensor cover 10 in an embodiment of the invention.

Fig. 4 is an expanded view of an example of fine holes provided on the surface of cover main body 14. Fig. 5A-5C is a diagram which schematically compares the operation of a conventional sensor cover with the operation of a sensor cover in an embodiment of the invention.

0028 As described above, a large number of fine holes are formed on the entire surface of the cover main body 14 portion of a sensor cover 10 according to an embodiment of the present invention, such that surrounding solution is able to flow into the interior of cover main body 14. In the sensor cover 10 of the present embodiment, the size of each fine hole formed on the lower surface of cover main body 14 and the size of each fine hole formed in the upper surface thereof are different. I.e., a lower transmitting portion 14a is provided for passing solution to the lower surface of cover main body 14, and an upper transmitting portion 14b is provided for passing solution to the upper surface thereof, and fine holes formed on the lower transmitting portion 14a are formed to be smaller than the fine holes formed in the upper transmitting portion 14b.

0029 As shown in Fig. 2, lower transmitting portion 14a is formed on the entire surface of the lower semicircular portion corresponding to the lower semicircle on cover main body 14, which has a circular cross section, and upper transmitting portion 14b is formed on the entire surface of the upper semicircular portion corresponding to the upper semicircle on cover main body 14. A large number of the same fine holes as are in lower transmitting portion 14a are also formed on the entire surface of cover main body 14 end surface 14c.

0030 Note that in this Specification, the "lower surface" of cover main body 14 means the surface on which light impinges when light is illuminated from vertically below onto sensor cover 10, shown installed in a state of use, and "upper surface" means the surface on which light falls when illuminated from vertically above. In the present embodiment a large number of fine holes are formed in the entirety of the "lower surface" and "upper surface" of cover main body 14, but it is not necessarily required that fine holes be formed on the entire surface; they may be formed in a portion only. In addition, in the present embodiment fine holes are also provided on the end surface 14c of cover main body 14, but it is also acceptable not to provide fine holes on the end surface 14c.

0031 Next, as shown in Fig. 4, approximately circular fine holes are arrayed in a staggered pattern on lower transmitting portion 14a and upper transmitting portion 14b. I.e., each fine hole is arrayed so that lines connecting the centers of three adjacent fine holes form an equilateral triangle. In the present embodiment, lower transmitting portion 14a and upper transmitting portion 14b are constituted by forming a large number of fine holes by etching into thin stainless steel plate. As an example, in the present embodiment, lower transmitting portion 14a is formed by arraying round holes with a diameter D = approximately 180µm at a pitch P = approximately 320µm (the length of the sides of an equilateral triangle connecting the centers of the circles). In addition, upper transmitting portion 14b is formed, for example, by arraying round holes with a diameter D = approximately 850µm at a pitch P = approximately 1270µm, or arraying round holes with a diameter D = approximately 350µm at a pitch P = approximately 630µm.

0032 Here, a lower transmitting portion 14a, in which round holes with a diameter D = approximately 180µm are arrayed at a pitch P = approximately 320µm, has a porosity of 28.7%, which roughly corresponds to an 80# mesh (80 mesh: a mesh in which 80 lines per inch are arrayed horizontally and vertically). In addition, upper transmitting portion 14b, in which round holes with a diameter D = approximately 850µm are arrayed at a pitch P = approximately 1270µm has a porosity of 40.6%, which roughly corresponds to a 20# mesh. An upper transmitting portion 14b, in which round holes with a diameter D = approximately 350µm are arrayed at a pitch P = approximately 630µm, has a porosity of 28%, which roughly corresponds to a 40# mesh.

Note that when round holes with a diameter D = approximately 100µm are arrayed at a pitch P = approximately 210µm, porosity is approximately 20.5%, which corresponds to a 150# mesh, and when round holes with a diameter D = approximately 150µm are arrayed at a pitch P = approximately 250µm, porosity is approximately 32.5%, roughly corresponding to a 100# mesh.

0033 Note that in the present embodiment, lower transmitting portion 14a and upper transmitting portion 14b are constituted by forming a large number of fine holes in a thin plate, but lower transmitting portion 14a or upper transmitting portion 14b may also be constituted by weaving or knitting to combine fine strands into a mesh. In this case the "fine holes" would be formed as the spaces between the strands constituting the mesh, and the "fine hole diameter" would mean the distance between adjacent strands. The diameter of fine holes provided in lower transmitting portion 14a is preferably set between approximately 100µm and 400µm. The diameter of fine holes provided in lower transmitting portion 14a is more preferably set between approximately 150µm and 400µm. To facilitate discharge of gas bubbles which enter into the cover main body, fine holes disposed on the upper transmitting portion 14b are preferably formed to be larger than the diameter of the fine holes on the lower transmitting portion 14a. However, it is desirable to set the diameter of the fine holes in upper transmitting portion 14b so that gas bubbles can be sufficiently suppressed from flowing in through upper transmitting portion 14b as the result of downward solution flow. The diameter of fine holes placed on upper transmitting portion 14b is preferably set to between approximately 1.5 times and approximately 5 times the diameter of the fine holes on lower transmitting portion 14a. This allows an appropriate balance between the suppression of gas bubble penetration into cover main body 14 and the discharge of those gas bubbles which do penetrate into cover main body 14.

0034 Next, referring to Fig. 5A-5C, we schematically explain the operation of a sensor cover.

First, a method has been known for some time whereby the effect of gas bubbles is reduced by covering a sensor with a mesh-shaped strainer when measuring properties of a solution into which gas bubbles are mixed. However with conventional covers of this type, as shown in Fig. 5A, if a coarse setting is used for the mesh opening of the strainer used to cover the sensor, large gas bubbles and small gas bubbles in the solution can easily penetrate into the cover, such that the influence of gas bubbles cannot be sufficiently suppressed.

0035 On the other hand, as shown in Fig. 5B, large gas bubbles can be prevented from penetrating if a fine setting is used for the mesh opening of a strainer covering a sensor. However a portion of small gas bubbles do penetrate into the cover. If a fine strainer mesh opening is set, gas bubbles which penetrate into the cover will have difficulty discharging from the interior of the cover, and will accumulate for a long time period inside the cover. Since gas bubbles accumulated in the cover float upward and accumulate in the upper portion of the cover, they will join together there to form large gas bubbles which are even more difficult to discharge from within the cover. For this reason, in conventional sensor covers many gas bubbles accumulated within covers as time elapsed, even when a fine strainer mesh opening was adopted, and these gas bubbles adversely influenced sensor measurement.

0036 By contrast, as shown in Fig. 5C, in the sensor cover 10 according to an embodiment of the present invention, small diameter fine holes are formed on the lower transmitting portion 14a of cover main body 14 so that penetration of large gas bubbles floating up from below into the cover main body 14 is stopped.

0037 On the other hand, if gas bubbles approach from below, small bubbles among them penetrate into cover main body 14 through lower transmitting portion 14a. However, large diameter fine holes are formed on upper transmitting portion 14b, therefore small gas bubbles which penetrate through lower transmitting portion 14a float up within cover main body 14 and are easily discharged to the outside of cover main body 14 through upper transmitting portion 14b. Therefore the accumulation of penetrating gas bubbles inside cover main body 14 over a long time period, and resultant growth into large gas bubbles, can be stopped.

0038 Gas bubbles which approach sensor cover 10 do not all approach from below, but because buoyancy from the solution is always acting on the gas bubbles, the fraction of gas bubbles approaching sensor cover 10 from below is high, and the penetration of these gas bubbles is greatly reduced by the lower transmitting portion 14a. In addition, penetration by some of the gas bubbles which approach sensor cover 10 from the side, etc. is also stopped by upper transmitting portion 14b.

0039 Because cover main body 14 is disposed to project diagonally downward, gas bubbles penetrating into cover main body 14 move upward to the base end portion of cover main body 14. On the other hand, the measurement portion 12b of the sensor probe 12a disposed inside cover main body 14 is disposed close to the end portion of cover main body 14, such that gas bubbles inside cover main body 14 are moved so as to travel away from the sensor 12 measurement portion 12b. Thus the influence on measurement by gas bubbles which have penetrated into cover main body 14 can be further reduced.

By the above actions, the influence of gas bubbles on measurement is effectively reduced using the sensor cover 10 of the present embodiment.

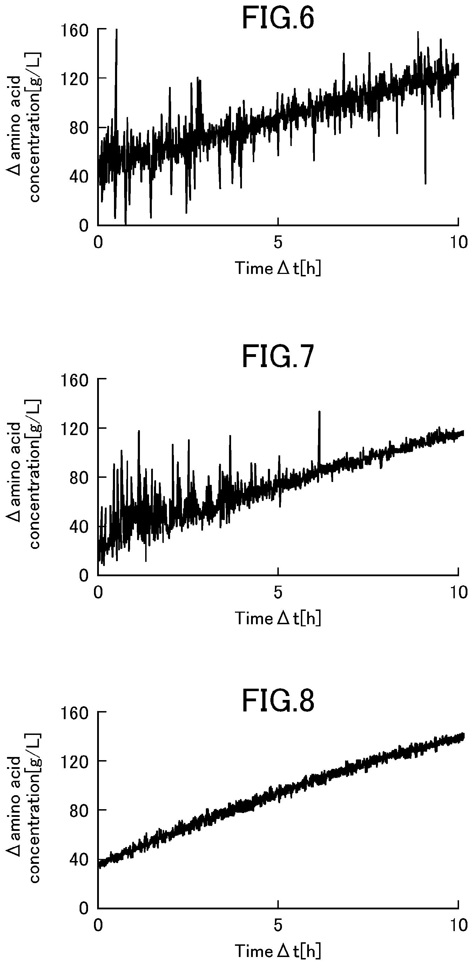

0040 Next, referring to Figs. 6 through 10, we explain an example of amino acid concentration measurement using a sensor apparatus 1 comprising a sensor cover 10 according to an embodiment of the present invention.

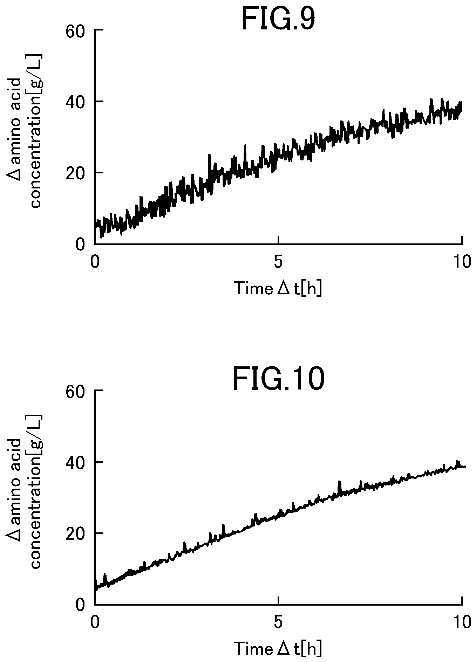

Fig. 6 shows an example of the case in which amino acid concentration is measured without using a sensor cover 10; Fig. 7 shows an example of the case in which amino acid concentration is measured using a conventional sensor cover (strainer). Fig. 8 is a diagram showing an example of amino acid concentration measurement by a sensor apparatus using a sensor cover according to an embodiment of the present invention. In addition, Figs. 9 and 10 are diagrams showing examples of respectively measuring the concentration of amino acid in a culture solution different from that of Figs. 6 through 8 using two types of sensor covers according to embodiments of the present invention.

0041 First, as described above, in the present embodiment sensor 12 is an NIR sensor, and light received at sensor probe 12a is guided to measurement instrument main unit 4 (Fig. 1) through an optical fiber. The NIR spectrum of the guided light is acquired by a measurement instrument main unit 4. The concentration of amino acid contained in the culture solution L can be estimated by applying a calibration model prepared in advance to the NIR spectrum obtained for the light from sensor probe 12a. Figs. 6 through 8 are graphs showing changes over time in the amino acid concentration estimated in this way.

0042 Fig. 6 is an example in which the amino acid concentration of culture solution L in fermenter 2 is measured (estimated) using sensor 12 without attaching a sensor cover 10. As shown in Fig. 6, while the trend of a rising concentration of amino acid in fermenter 2 over time can be seen in the measurement conducted without using sensor cover 10, there is an extremely large variability in the estimated values for each measurement. This variability is believed to occur because gas bubbles mixed into the culture solution L affect the light received at sensor probe 12a.

0043 Fig. 7 shows an example in which the concentration of amino acid in the culture solution L measured in the example shown in Fig. 6 is measured using a sensor 12 covered by a 20# strainer (a conventional sensor cover). As shown in Fig. 7, compared to the Fig. 6 example the variability in amino acid concentration estimated values is reduced as a result of covering sensor 12 with a strainer. However, there is a time period when estimated values vary greatly even when a strainer is used, and with a conventional sensor cover the amino acid concentration cannot be estimated with sufficient reliability.

0044 Fig. 8 is an example in which the concentration of amino acid in culture solution L measured in the Fig. 6 and 7 example is measured using a sensor cover 10 according to an embodiment of the present invention. Note that in the Fig. 8 example, a cover main body 14 is used in which a mesh with approximately 180µm round holes arrayed at a pitch of approximately 320µm is provided as the lower transmitting portion 14a, and a mesh with approximately 850µm round holes arrayed at a pitch of approximately 1270µm is provided as the upper transmitting portion 14b. As shown in Fig. 8, variability of the estimated values of amino acid concentrations is extremely small compared to the examples shown in Figs. 6 and 7, as a result of covering sensor 12 with the sensor cover 10 of the present embodiment, such that sufficiently reliable estimated values can be obtained.

0045 Next, referring to Figs. 9 and 10, we explain an example of estimating the concentration of amino acid in fermenter 2, in which an amino acid different from that of the Fig. 6 through 8 examples is being cultured.

Here an aerobic culture is cultured in the fermenter 2 used in the amino acid concentration estimation examples of Figs. 6 through 8, and fine gas bubbles with a diameter of approximate l50µm are introduced into culture solution L by a supply apparatus (not shown). In contrast, no gas bubbles are introduced into the fermenter 2 shown in the Fig. 9 and 10 measurement examples, and the gas bubbles which are mixed in are primarily fine gas bubbles with a diameter of approximately 350µm resulting from carbon dioxide gas produced in culture solution L.

0046 Fig. 9 is an example in which the amino acid concentration of culture solution L is measured using a sensor cover 10 according to an embodiment of the present invention. Note that in the Fig. 9 example, as in the Fig. 8 example, a cover main body 14 is used in which a mesh with approximately 180µm round holes arrayed at a pitch of approximately 320µm is provided as the lower transmitting portion 14a, and a mesh with approximately 850µm round holes arrayed at a pitch of approximately 1270µm is provided as the upper transmitting portion 14b. As shown in Fig. 9, variability of the estimated amino acid concentration values is kept low by covering sensor 12 with the sensor cover 10 of the present embodiment, so that highly reliable estimated values are obtained.

0047 Fig. 10 is an example in which the concentration of amino acid in the same culture solution L as the Fig. 9 example is measured using a sensor cover 10 according to an embodiment of the present invention. Note that in the Fig. 10 example a cover main body 14 is used in which a mesh with approximately 180µm round holes arrayed at a pitch of approximately 320µm is provided as the lower transmitting portion 14a, and a mesh with approximately 350µm round holes arrayed at a pitch of approximately 630µm is provided as the upper transmitting portion 14b. In the Fig. 10 example, as well, variability in the estimated amino acid concentration values is kept extremely low, and sufficiently high reliability is obtained.

0048 In the example shown in Fig. 10, variability in estimated values is even lower than the Fig. 9 example. Thus the effects from mixing in of gas bubbles can be sufficiently suppressed by respectively setting the size of the fine holes formed in the lower transmitting portion 14a and upper transmitting portion 14b according to properties such as the nature of the gas bubbles mixed into the solution being measured, or the viscosity of the solution.

0049 Using a sensor cover 10 according to an embodiment of the present invention, a lower transmitting portion 14a and upper transmitting portion 14b for passing solution are formed on a cover main body 14 disposed to a surround sensor 12 (Fig. 2), therefore the solution to be measured is able to flow constantly in and out of cover main body 14. As a result, solution in the cover main body 14 is constantly exchanged, and a sensor 12 disposed inside the cover main body 14 can continuously measure properties of the solution in real time (Figs. 8 through 10). Also, the fine holes in lower transmitting portion 14a are formed to be smaller than the fine holes in upper transmitting portion 14b (Fig. 5C), therefore gas bubbles floating up from below cover main body 14 have difficulty passing through the fine holes in lower transmitting portion 14a, and penetration of gas bubbles into cover main body 14 can be effectively suppressed. At the same time, fine holes in upper transmitting portion 14b are formed to be larger than the fine holes in lower transmitting portion 14a, so gas bubbles penetrating into cover main body 14 float up within the cover main body and are easily discharged through upper transmitting portion 14b. As a result, gas bubbles penetrating into cover main body 14 can be suppressed from accumulating in cover main body 14 and adversely influencing sensor 12 measurement values.

0050 Using a sensor cover 10 according to the present embodiment, cover main body 14 is formed in an essentially cylindrical shape (Fig. 2), therefore gas bubbles floating up from the lower side of cover main body 14 can flow easily along the lower surface of cover main body 14, and penetration of gas bubbles into cover main body 14 can be further suppressed. Since cover main body 14 is formed as an essentially cylindrical shape, gas bubbles which have penetrated into cover main body 14 and floated up are collected at the highest part within cover main body 14 and have difficulty contacting sensor 12. Thus even if gas bubbles penetrate into cover main body 14, their adverse influence on measurement can be minimized.

0051 Also, in the sensor cover 10 of the embodiment, the lower transmitting portion 14a and upper transmitting portion 14b are respectively provided on the entire surface of the lower semicircular portion and upper semicircular portion thereof (Fig. 2), so the part of the cover main body 14 through which solution passes can be made extremely large. As a result, exchange of solution inside the cover main body 14 with solution outside occurs more easily, and properties of the solution can be accurately measured in real time.

0052 Also, with the sensor cover 10 of the present embodiment, the fine holes in lower transmitting portion 14a and upper transmitting portion 14b are formed by holes made in thin metal plate (Fig. 4), therefore in comparison to the case in which a lower transmitting portion or upper transmitting portion are formed by a mesh made by weaving strands, solution is less prone to adhere to lower transmitting portion 14a and upper transmitting portion 14b, and maintainability of the sensor cover 10 can be improved.

0053 In addition, with the sensor cover 10 of the present embodiment, the cover main body 14 projects diagonally downward from the side wall surface 2a of fermenter 2, which is a solution-holding container (Fig. 3), so gas bubbles floating up from below and reaching the cover main body 14 can easily flow upward along the bottom surface of cover main body 14, and penetration thereof into the cover main body 14 can be effectively suppressed. Gas bubbles which do penetrate into the cover main body 14 collect at the base end portion of the cover main body 14 positioned above, traveling away from the measurement portion 12b of sensor 12 positioned close to the end of cover main body 14, thereby reducing their influence on measurement.

0054 Above we have explained preferred embodiments of the present invention, but various changes may be added to the above-described embodiments. In particular, in the above-described embodiments of the invention the concentration of amino acid in a culture solution in which gas bubbles are mixed is estimated by spectral analysis, but the present invention may be applied to measure the properties of any desired solution in which gas bubbles are mixed. Also, in the above-described embodiment the invention was applied to measure the concentration of particular components in a solution, but the invention may also be applied to a sensor cover for any desired sensor used to measure properties of a solution itself, such as dielectric constant, conductivity, and the like.

0055 In the above-described embodiment of the invention, the invention was applied to the measurement of amino acid concentration in a culture solution, but an aerobic culture can be accurately controlled by applying the invention to the measurement of various component concentrations not limited to amino acid concentration, thereby increasing the accumulation and yield of products in the culture solution. For example, by applying the invention to the measurement of carbon source concentration as set forth in Patent Document 1 described above, carbon source concentration can be measured in-line.

### Explanation of Reference Numerals

0056
1: sensor apparatus
2: fermenter
2a: side wall surface
4: measurement instrument main unit
6: mixer
6a: blades
10: sensor cover
12: sensor
12a: sensor probe
12b: measurement portion
14: cover main body
14a: lower transmitting portion
14b: upper transmitting portion
14c: end surface
16: flange portion
18a: affixing bolt
18b: packing

## Claims

1. A sensor cover for a sensor for measuring properties of a solution into which gas bubbles are mixed, comprising:
a cover main body disposed to surround the sensor;
wherein a lower transmitting portion for passing solution is provided on at least a portion of a lower surface of the cover main body, and an upper transmitting portion for passing solution is provided on at least a portion of an upper surface of the cover main body; and
a large number of fine holes for passing solution are respectively formed on the lower transmitting portion and the upper transmitting portion, wherein the fine holes provided on the lower transmitting portion are formed to be smaller than the fine holes provided on the upper transmitting portion.

2. The sensor cover of Claim 1, wherein the cover main body is formed in an approximately cylindrical shape, and the sensor extends in an axial direction on an interior of the cover main body.

3. The sensor cover of Claim 2, wherein the lower transmitting portion is placed on an entire surface of a lower semicircular portion of the approximately cylindrical cover main body, and the upper transmitting portion is placed on an entire surface of an upper semicircular portion of the cover main body.

4. The sensor cover of any one of Claims 1 through 3, wherein the cover main body is formed of a thin metal plate, and the fine holes in the lower transmitting portion and the upper transmitting portion are approximately round holes made in the thin metal plate.

5. The sensor cover of any one of Claims 1 through 4, wherein a diameter of the fine holes provided in the upper transmitting portion is between 1.5 and 5 times a diameter of the fine holes provided in the lower transmitting portion.

6. The sensor cover of any one of Claims 1 through 5, wherein a diameter of fine holes in the lower transmitting portion is between 100µm and 400µm.

7. The sensor cover of any one of Claims 1 through 6, wherein the cover main body is placed to project diagonally downward from a side wall surface of a container in which the solution is held, and a measurement portion of the sensor is disposed close to an end of the cover main body.

8. A sensor apparatus comprising:
a sensor for measuring properties of a solution; and
the sensor cover of any one of Claims 1 through 7, disposed to surround the sensor.

9. A method for measuring properties of a solution, the method comprising:
a step for preparing the sensor apparatus of Claim 8; and
a step for acquiring a measurement signal from the sensor apparatus inserted into the solution.

10. A method for manufacturing metabolic products in an aerobic culture, the method comprising:
a step for preparing the sensor apparatus of Claim 8;
a step for acquiring a measurement signal from the sensor apparatus inserted into a culture solution;
a step for aerobic culturing in the culture solution; and
a step for tracking changes in concentration of the metabolic products in the aerobic culture and/or changes in concentration of a culture raw material based on the measurement signal.
